# EUROPEAN PATENT APPLICATION

(11) **EP 3 566 723 A1**
(43) Date of publication of application: **13.11.2019**
(21) Application number: 18382323.6
(22) Date of filing: 11.05.2018
(51) Int. Cl.: A61L 2/20

(54) **METHOD AND SYSTEM FOR NEUTRALIZING FORMALDEHYDE**

(71) Applicant: Anathomic Solutions, S.L., 08223 Terrassa (ES)
(72) Inventor: MARTÍNEZ CLOSAS, Mª Antonia, 08223 TERRASSA (ES); GONZÁLEZ PRIMO, Francisco, 08224 TERRASSA (ES)
(74) Representative: Sugrañes Patentes y Marcas

(57) **Abstract**

Method for neutralizing formol, especially suitable for neutralizing formol used in centers for analyzing biological tissues, characterized in that it comprises the operations of:
a) supplying and storing quantities of formol to be neutralized associated with various uses or analyses in at least one reaction vessel (3, 4);
b) stopping the supply of formol to be neutralized when a maximum fill level of the formol content inside the reaction vessel (3, 4) has been reached in order to initiate the neutralization process;
c) supplying an ozone current inside the reaction vessel (3, 4) to produce a mixture with the formol content during a pre-established time to generate the neutralization process of the formol;
d) stopping the supply of the ozone current when the neutralization process of the formol has finished; and
e) draining the content of neutralized formol from the reaction vessel (3, 4) for its disposal.

## Description

### Technical sector of the invention

The invention relates to a method for neutralizing formol, especially suitable for neutralizing formol used in centers for analyzing biological tissues, such as pathological anatomy laboratories, hospitals, autopsy rooms, toxicology, thanatopraxy, legal medicine, universities, etc.

The invention also relates to a system for neutralizing formol, especially suitable for performing said method.

"Formol used" is understood here as formol which is disposed of after carrying out laboratory work requiring the use of formol, such as biopsies, perfusions, tissue washes or similar.

"Neutralized formol" is understood here as the water and carbon dioxide-based aqueous solution obtained by a chemical reaction by means of which the formol is completely decontaminated.

### Background of the invention

As is known, formol is used as a preservative of biological samples, tissues and to preserve cadavers, therefore being commonly used in centers such as pathological anatomy laboratories, hospitals, autopsy rooms, toxicology, thanatopraxy, legal medicine, universities, etc.

Formol is the substance formed by the aqueous solution of formaldehyde (HCHO), commonly 37 to 50%, formaldehyde being a colorless gas with a strong and unpleasant odor, very soluble in water. However, formaldehyde is considered a potentially carcinogenic substance.

In fact, there are studies carried out in anatomy and pathological anatomy laboratories which reveal that during the works in which formol is used (biopsies, perfusions, tissue washes, etc.), high levels of environmental contamination are produced by the emissions of formaldehyde. As a result, the persons carrying out the laboratory work are seriously affected by the emissions of this contaminant, causing serious health problems such as ocular damage, throat irritations, allergic reactions to the skin, nausea, asphyxia, amongst others.

At present, rigorous environmental control of this contaminant substance is required to guarantee the required safety and hygiene conditions of the operators of these centers where formol is used.

At present, there are trimming stations or tables for carrying out operations with formol (biopsies, tissue washes, etc.) which are equipped with tools and components necessary for carrying out these tasks and which also incorporate safety elements, such as air extraction screens or hoods, which enable compliance with the safety and hygiene regulations of the workers.

Additionally, these trimming tables have a sink for collecting formol used with a drain connected to a replaceable vessel or drum, where the quantities of formol used associated with various uses or analyses will be stored. Once the vessel has been filled, the next step is its subsequent disposal management since the used formol cannot be disposed of directly into the general sewerage network since it is a contaminant substance.

For years, it has been observed that the disposal of the used formol is a real problem for the centers where formol is used, therefore an effective solution is required in this respect.

At present, the disposal of the used formol by these centers can be carried out in two different ways:
1) By contracting external companies specialized in the disposal of contaminant residues. However, contracting this type of service involves an increased cost for these centers, in addition to being required to have a small collection store for the vessels of used formol.
2) Dissolving a specific chemical product, known as Scigen®, to neutralize the formol. However, in practice, it has been demonstrated that the use of this product has consistent problems, such as:
   - It is a product that is very difficult to apply since it produces solid residues.
   - This manner of neutralizing the formol requires constant cleaning as its form of application is not efficiently achieved, therefore the technical personal who carry out these tasks are also exposed to inhalations which, as previously mentioned, are harmful to health.
   - The cost involved in the constant maintenance required by the installation would have to be added to the high economic cost of the product.
   - This product is considered for the absorption and neutralization of formol in accidental occasional spills, not for carrying out continuous work.
   - Another problem with this system is the control of the pH neutralized solution since it should be carried out manually, therefore being exposed to inhalations, also generating a new economic cost since there always has to be a large number of pH control kits available.

At present, due to the inefficiency and problems exhibited by said chemical product for neutralizing the formol, some centers that have used this product have decided to end its application.

It would therefore be desirable to have an optimal solution allowing the used formol to be efficiently neutralized in these types of centers without any kind of risk to health and which also guarantees its disposal without damage to the environment.

It is also interesting that the solution can be implemented in a structurally simple and economic manner without the need to use consumables, nor chemical additives.

### Description of the invention

With the aim of providing a solution to the problems posed, a method for neutralizing formol is made known, especially suitable for neutralizing formol used in centers for analyzing biological tissues, characterized in that it comprises the operations of:
a) supplying and storing quantities of formol to be neutralized associated with various uses or analyses in at least one reaction vessel;
b) stopping the supply of formol to be neutralized when a maximum fill level of the formol content inside the reaction vessel has been reached in order to initiate the neutralization process;
c) supplying an ozone current inside the reaction vessel to produce a mixture with the formol content during a pre-established time to generate the neutralization process of the formol;
d) stopping the supply of the ozone current when the neutralization process of the formol has finished; and
e) draining the content of neutralized formol from the reaction vessel for its disposal.

The quantities of formol to be neutralized associated with various uses or analyses are therefore going to be stored temporarily in the corresponding reaction vessel. Once the reaction vessel has reached its maximum fill level, the neutralization process with ozone will be initiated.

The ozone supply will preferably be carried out by injecting a continuous ozone current. Alternatively, the ozone application could be carried out by means of injecting intermittent bursts, for example every second or every half second, depending on the needs.

Formol is the substance formed by the aqueous solution of formaldehyde (HCHO). It should be pointed out ozone (O₃) can catalytically oxide formol to obtain water (H₂O) and carbon dioxide (CO₂), clean reaction products since the carbon dioxide evaporates.

In this way, optimal neutralization of the formol used is achieved in centers for analyzing biological tissues such as pathological anatomy laboratories, hospitals, autopsy rooms, toxicology, thanatopraxy, legal medicine, universities, etc., since the neutralized formol can be drained directly into the general sewerage network, completely decontaminated. This guarantees compliance with environmental regulations in force, thereby avoiding the contracting of an external service to dispose of residues, with the consequent reduction of costs.

In addition, possible risks to the health of the operators carrying out these works with formol are avoided, since it is not necessary to handle the formol to be neutralized during the method, nor is it necessary to add any type of chemical product to carry out the neutralization, unlike what would occur in the methods known in the prior art.

Moreover, the costs of maintenance will be notably reduced since the use of consumables, or chemical additives is not necessary.

Preferably, the method comprises the operation of stirring the formol content inside the reaction vessel while the ozone current is supplied to facilitate its mixing during the neutralization reaction.

Advantageously, the method comprises the operation of measuring the pH of the formol and ozone mixture such that before carrying out the draining operation, it is verified whether the pH value of the neutralized formol is within standardized set values for decontamination of the formol, established at 7 pH (pH neutral).

The measurement of the pH will preferably be carried out continuously. However, it is also envisaged that said measurement of the pH is carried out at certain pre-established time periods or intervals during the neutralization process.

Preferably, the flow of formol to be neutralized is subjected to a solid filtering operation before being supplied to the reaction vessel. In this way, the accumulation of solid residues in the interior of the reaction vessel is avoided.

According to a preferred embodiment of the invention, the operation of supplying a flow of formol to be neutralized is carried out continuously by means of using two reaction vessels arranged in parallel which work in alternating cycles, such that while a first reaction vessel is carrying out the neutralization reaction of the formol, the second reaction vessel is storing formol to be neutralized and vice versa, thereby guaranteeing continuous functioning of the method.

According to another aspect, the invention also relates to a system for neutralizing formol, especially suitable for neutralizing the formol used in centers for analyzing biological tissues, characterized in that it comprises
- an intake of quantities of formol to be neutralized associated with various uses or analyses;
- at least one reaction vessel which comprises
   - an inlet of the flow of formol to be neutralized connected to said intake of the system,
   - a fill level detector of the formol content to be neutralized inside the reaction vessel, intended to send a signal to control means to initiate the neutralization process when a maximum fill level has been reached,
   - a supply inlet of an ozone current, intended to produce a mixture with the formol content to generate the neutralization process of the formol,
   - an outlet of the flow of neutralized formol;
- at least one ozone generator equipped with an outlet for the injection of said ozone current towards the interior of the at least one reaction vessel; and
- a drain connected to the outlet of the at least one reaction vessel to remove the neutralized formol out of the system.

The system of the invention allows the formol used to be neutralized by means of the use of ozone in a structurally simple and effective manner. In effect, as has already been mentioned, the neutralized formol can be drained directly into the general sewerage network, completely decontaminated, guaranteeing compliance with the environmental regulations in force, and without risk to the health of the operators since it is not necessary to be in contact with the formol which is going to be neutralized, nor to handle chemical products to carry out the neutralization.

Additionally, it should be pointed out that the use of an ozone generator, capable of transforming the oxygen from the air into ozone allows the maintenance costs to be reduced considerably since the use of consumables, or chemical additives is not necessary.

According to a preferred embodiment of the invention, the system comprises two reaction vessels intended to work in alternating cycles, both vessels being connected in parallel to the intake, to the outlet of the ozone generator and to the drain, by way of diverting conduits in the manner of bypasses, controlled by respective electrovalves. In this way, the continuous functioning of the system is guaranteed.

According to one characteristic of the invention, the reaction vessel, or when applicable each reaction vessel, also comprises a motor for actuating rotary blades configured to stir the formol content inside the respective reaction vessel and facilitate its mixing with the ozone during the neutralization process.

Advantageously, the reaction vessel, or when applicable each reaction vessel, also comprises a pH measurer intended to monitor the pH level of neutralized formol before proceeding to its drainage. Said pH measurer is arranged for safety to thereby guarantee the complete decontamination of the formol before proceeding to its drainage.

Preferably, the system comprises a filter arranged before the reaction vessel, or when applicable the reaction vessels, intended to prevent the accumulation of solid residues in its interior.

According to another characteristic of the invention, the system comprises a pump intended to force the outlet of the content of neutralized formol towards the drain.

Advantageously, the system comprises a surrounding casing configured to house in its interior all the components of the system in a compact manner.

Additionally, the system comprises a sampling tap arranged before the drain, intended to take samples of the neutralized formol for its analysis.

According to one characteristic of the invention, the drain of the neutralized formol comprises means for its connection to the general sewerage network.

According to another characteristic of the invention, the intake of the formol to be neutralized comprises means for its connection to an exterior conventional drain for collecting formol.

### Brief description of the drawings

In the attached drawings, a preferred embodiment of the system for neutralizing formol of the invention is illustrated in an exemplary and non-limiting manner. In said drawings:
Fig. 1 is a schematic diagram of the system of the invention according to a preferred embodiment; and
Figs. 2 to 8 show a sequence of the steps of functioning of the system of Fig. 1 to carry out the method of neutralizing the formol.

### Detailed description of an embodiment of the invention

The system 1 for neutralizing formol according to the invention is especially suitable for neutralizing formol used in centers for analyzing biological tissues, such as pathological anatomy laboratories, hospitals, autopsy rooms, toxicology, thanatopraxy, legal medicine, universities, etc.

As has been mentioned in the section on the background of the invention, these types of centers use trimming tables to carry out different works with formol (biopsies, tissues washes, etc.) which are equipped with a sink with a drain for collecting the used formol.

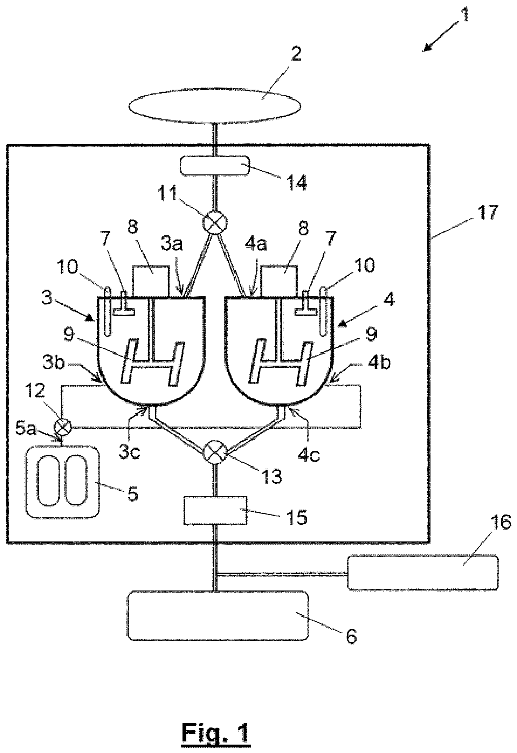

The system 1 of the invention, shown schematically in Figure 1, according to a preferred embodiment, comprises:
- an intake 2 of the quantities of formol used to be neutralized associated with various uses or analyses;
- two reaction vessels 3, 4, arranged in parallel connected respectively to said intake 2 which work in alternating cycles such that while a first reaction vessel 3 is carrying out the neutralization reaction of the formol, the second reaction vessel 4 is storing the formol to be neutralized and vice versa, to guarantee continuous functioning of the system, as will be explained below;
- an ozone generator 5 capable of transforming the oxygen (O₂) from the air into ozone (O₃) provided with an outlet 5a for the injection of an ozone current towards the interior of each reaction vessel 3, 4 such that the mixture of formol and ozone in the interior of the respective reaction vessel 3, 4 produces the neutralization reaction of the formol; and
- a drain 6 connected respectively to each reaction vessel 3, 4 to remove the neutralized formol out of the system.

Therefore, in the corresponding reaction vessel 3, 4, the quantities of formol to be neutralized associated with various uses or analyses will be temporarily stored, by way of the intake 2. In this example, the intake 2 will be connected externally to the respective drain of the sinks for collecting the formol used, present in the trimming tables known in the prior art. Once the reaction vessel 3, 4 reaches its maximum fill level, the neutralization process with ozone will be initiated.

Formol is the substance formed by the aqueous solution of formaldehyde (HCHO). Ozone (O₃) can catalytically oxide formol to obtain water (H₂O) and carbon dioxide (CO₂), clean reaction products since the carbon dioxide evaporates.

The system 1 of the invention allows the formol used to be neutralized by means of the use of ozone in a structurally simple and effective manner. In effect, as has already been mentioned, the neutralized formol can be drained directly into the general sewerage network, completely decontaminated, guaranteeing compliance with the environmental regulations in force, and without risk to the health of the operators since it is not necessary to be in contact with the formol which is going to be neutralized, nor to handle chemical products to carry out the neutralization.

Additionally, the use of an ozone generator 5, capable of transforming the oxygen from the air into ozone allows the maintenance costs to be reduced considerably since the use of consumables, or chemical additives is not necessary.

Although a single ozone generator 5 has been represented in the figures, the use of a double ozone generator is envisaged with alternative functioning to guarantee the effectiveness of the system in the event of failure of one of these.

In this example, it is envisaged that each reaction vessel 3, 4 has a capacity of 5 liters. It should be pointed out that the 5 liter capacity is more than sufficient to ensure neutralization under more unfavorable working conditions. Between 5 and 7 minutes will be sufficient for a vessel with a capacity of 5 liters to complete the neutralization cycle.

In addition, in this example, the supply of ozone is carried out by means of a preferably continuous ozone current and using a maximum flowrate of 10 g/h of ozone (O₃).

In this preferred embodiment, two identical reaction vessels 3, 4 are used, each one comprising the following components:
- an inlet 3a, 4a of the flow of formol to be neutralized connected to said intake 2 of the system 1;
- a fill level detector 7 of the formol content to be neutralized inside the reaction vessel 3, 4 capable of sending a signal to activate the neutralization process when the maximum fill level of the first reaction vessel 3 is reached, at the same time sending a signal so that the second reaction vessel 4 starts to fill;
- a supply inlet 3b, 4b of an ozone current coming from the ozone generator 5 provided for producing a mixture with the formol content to generate the neutralization process of the formol;
- a motor 8 for actuating rotary blades 9 configured to stir the formol content inside the respective reaction vessel 3, 4 and facilitate its mixing with the ozone during the neutralization reaction;
- a pH measurer 10, preferably continuous, arranged for security to monitor the pH level of the neutralized formol solution before it is drained. The pH value of the measured neutralized formol is compared with standardized set values established at 7 pH (pH neutral) to thereby guarantee the complete decontamination of the formol;
- an outlet 3c, 4c of the flow of neutralized formol connected to said drain 6 of the system 1.

Both vessels 3, 4 are connected in parallel to the intake 2, to the outlet 5a of the ozone generator 5 and to the drain 6, by way of diverting conduits in the manner of bypasses, controlled by respective electrovalves 11, 12 and 13.

In this case, a first electrovalve 11 is used to determine which of the two reaction vessels 3, 4 has to receive the inlet of formol to be neutralized; a second electrovalve 12 to determine which of the two reaction vessels 3, 4 has to receive the ozone current; and a third electrovalve 13 which controls the outlet of neutralized formol from the respective reaction vessels 3, 4.

The system 1 also comprises a filter 14 arranged before both reaction vessels 3, 4 intended to prevent the accumulation of solid residues in its interior.

The system 1 is also equipped with a pump 15 provided to force the outlet of the content of neutralized formol towards the drain 6.

Additionally, the system 1 includes a manually-controlled sampling tap 16 arranged before the drain 6 intended to take samples of the neutralized formol in the event that an external analysis is required.

The system 1 also comprises a surrounding casing 17 formed by a closed box which incorporates in its interior the two reaction vessels 3, 4, the ozone generator 5 and the rest of the components of the system in a compact manner, as can be seen in Figure 1. In this embodiment, the intake 2 of the formol to be neutralized is arranged in the upper part of the casing 17, while the drain 6 of the neutralized formol is located in the lower part of said casing 17 for its drainage by the effect of gravity.

The surrounding walls of the casing 17 have different perforated zones to ensure the circulation of air, thus achieving the ventilation of the internal components.

The casing 17 and the two reaction vessels 3, 4 are preferably made of stainless steel, other types of suitable materials being able to be optionally used.

The system 1 also comprises a screen (not represented) connected to computerized control means for managing the neutralization method as well as the different parameters which are involved, such as fill levels, alarms, electrovalves, etc., which guarantee continuous functioning without incidents. The installation of this control screen can be easily carried out by means of a multipolar connection to the external body of the casing 17.

Another advantage of the system 1 of the invention is its simple installation in the centers for analyzing biological tissues. In effect, for such purpose:
- The intake 2 of the formol to be neutralized comprises means for its external connection to the drain of the sinks for the collection of formol used, present in the conventional trimming tables of the centers for analyzing biological samples.
- The drain 6 of the neutralized formol is equipped with means for its connection to the general waste water sewerage network.
- The cited solid filter 14 is arranged adjacent to the intake 2 so as to be easy to remove to ensure the safety of the user.
- A tension connector (not represented) is arranged for taking current and connection to the corresponding electric network.

Another advantage is without doubt the simplicity of the method since, as has been mentioned, a few minutes is sufficient for completing the neutralization process of the reaction vessel 3, 4 before draining its content to the general sewerage network.

In addition to be a quick process, there is the guarantee that the system 2 will not stop working since the second reaction vessel 4 will be active for its filling while the first reaction vessel 3 is in the process of neutralizing and draining. The laboratory technician will therefore not have to stop his work while the method of neutralization is ongoing.

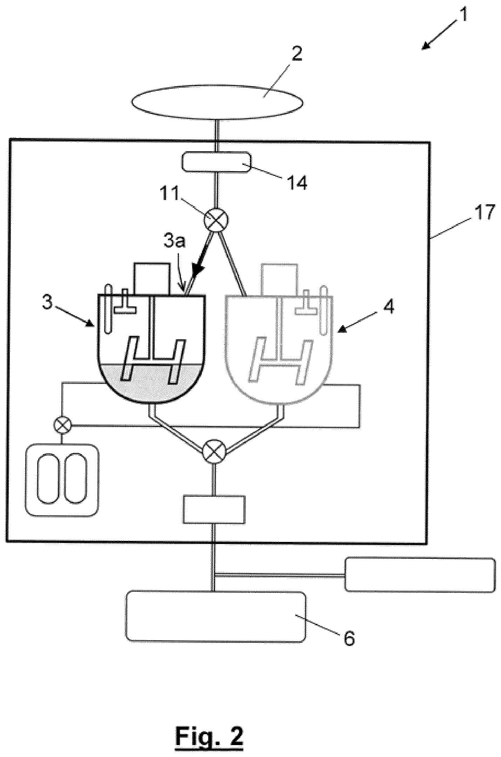

The method of neutralizing formol of the system of the invention will be explained below making reference to the figures 2 to 8.

As can be seen in Figure 2, the method starts with the filling of the first reaction vessel 3 with the quantities of formol used associated with various uses or analyses which are going to accumulate in the reaction vessel 3 through the intake 2. To this end, the first electrovalve 11 will allow the selection of the reaction vessel to be filled, the inlet 3a of the first reaction vessel 3 being activated in this case. It should be pointed out that while the first reaction vessel 3 is filled, the rest of its components are at rest (fill detector 7, motor 8 of the rotary mixers 9, pH detector 10, electrovalve 12 of ozone supply), awaiting its corresponding activation signal.

Therefore, at this time, the first reaction vessel 3 is active in the filling phase (represented by a continuous black line); while, the second reaction vessel 4 is empty in the inactive state (represented by a continuous gray line).

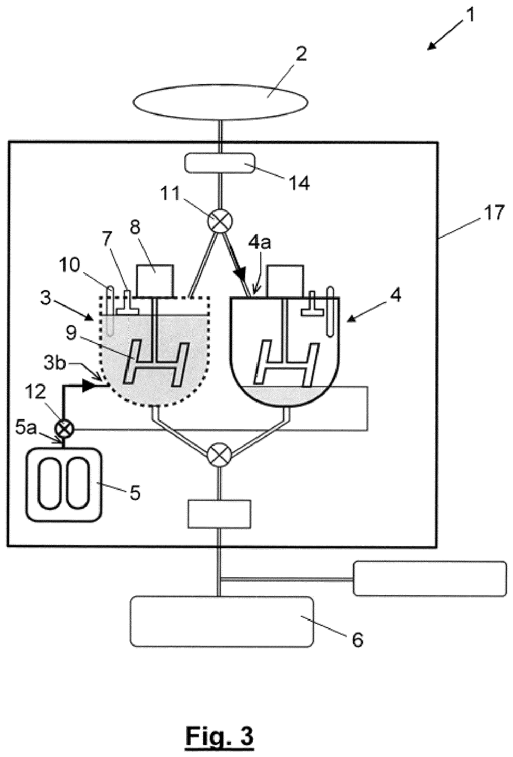

It can be observed in Figure 3 that once the first reaction vessel 3 reaches its maximum fill level, detected by means of its corresponding level detector 7, various simultaneous processes are generated:
- The inlet 4a is activated for the filling of the second reaction vessel 4; therefore, it goes to the active state (represented by a continuous black line).
- The ozone generator 5 is activated and the second electrovalve 12 allows the supply of the ozone current towards the first reaction vessel 3 (now represented by a dotted line) through its inlet 3b for carrying out the neutralization process.
- The motor 8 is activated which actuates the blades 9 of the first reaction vessel 3. The blades 9 move the entire formol content to guarantee the homogeneity of the ozonation of the content.
- The pH measurer 10 sends information to the control means to control the levels of the neutralized formol content of the first reaction vessel 3 before being drained.

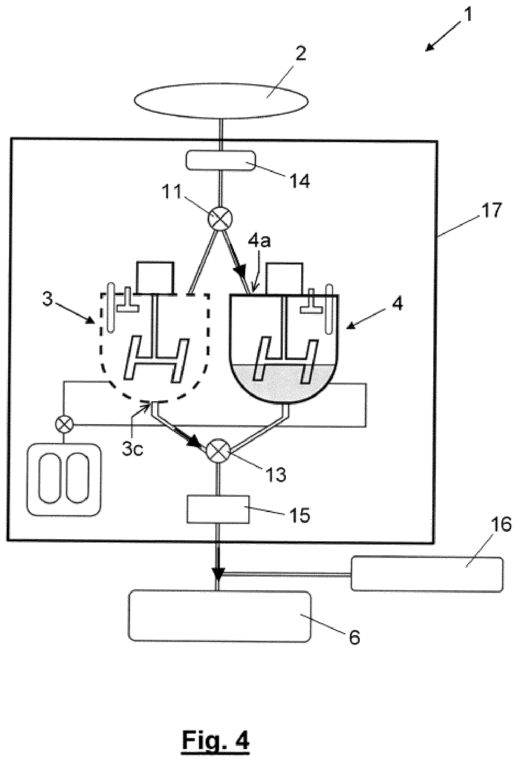

Then, as is shown in Figure 4, when the neutralization process has finished in the first reaction vessel 3 (now represented by a discontinuous dashed line), the third electrovalve 13 is activated which allows the removal of the content of neutralized formol through the outlet 3c of said first reaction vessel 3. At the same time, the pump 15 enters into operation to drive the content of neutralized formol from the first reaction vessel 3 to the drain 6 for its completely decontaminated removal towards the general sewerage network. At the same time, the second reaction vessel 4 continues to be filled (represented by a black continuous line).

In the event that a sample of neutralized formol is required for an external analysis, it could be taken from the sampling tap 16 arranged before the drain 6.

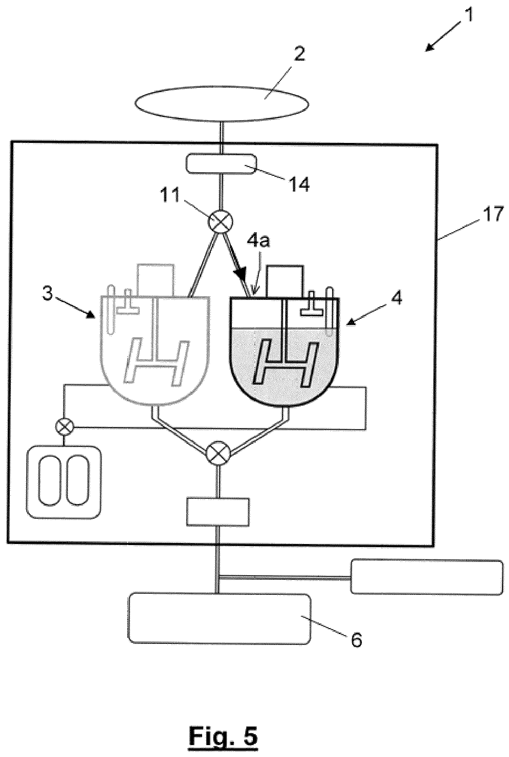

As can be observed in Figure 5, the first reaction vessel 3 is empty in the inactive state (represented by a continuous gray line). While the filling process of the second reaction vessel 4 continues (represented by a continuous black line), the rest of its components still being at rest (fill detector 7, motor 8 of the mixer blades 9, pH detector 10, electrovalve 12 of ozone supply), awaiting to receive the activation signal.

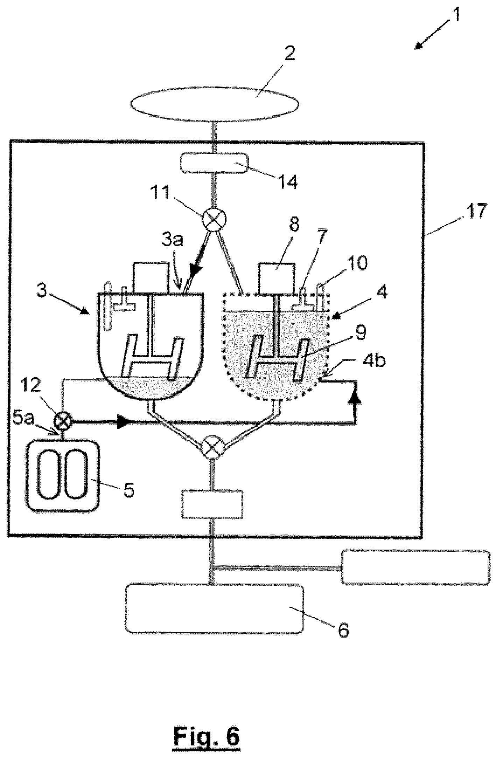

Now referring to Figure 6, it can be seen that the second reaction vessel 4 reaches its maximum fill level, detected by means of its corresponding level detector 7, the simultaneous process described above having started:
- The filling of the first reaction vessel 3 is activated by way of its inlet 3a, consequently it goes into the active state (represented by a continuous black line).
- The ozone generator 5 is activated and the second electrovalve 12 allows the supply of the ozone current towards the second reaction vessel 4 (now represented by a dotted line) through its inlet 4b to carry out the neutralization process.
- The motor 8 is activated which actuates the blades 9 of the second reaction vessel 4.
- The pH measurer 10 sends information to the control means to control the levels of the neutralized formol content of the second reaction vessel 4 before being drained.

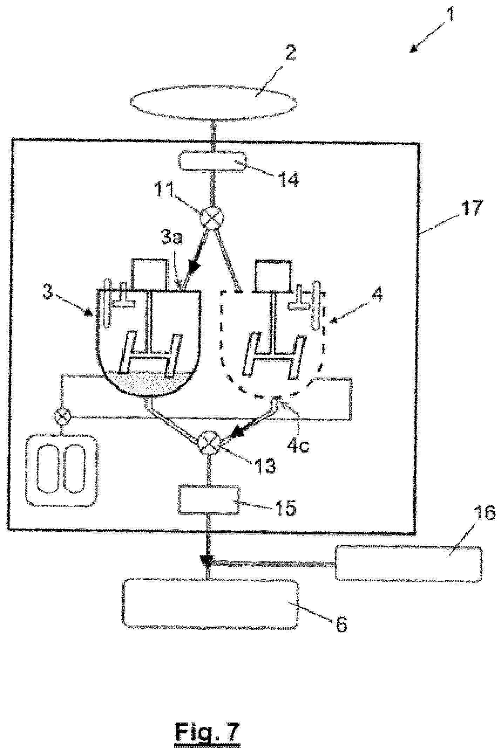

Subsequently, as can be observed in Figure 7, when the neutralization process has finished in the second reaction vessel 4 (now represented by a discontinuous dashed line), the third electrovalve 13 is activated which allows the removal of the content of neutralized formol through the outlet 4c of said second reaction vessel 4. At the same time, the pump 15 enters into operation to drive the content of neutralized formol from the second reaction vessel 4 to the drain 6 for its completely decontaminated removal towards the general sewerage network. At the same time, the first reaction vessel 3 continues to be filled (represented by a black continuous line).

In the same way, in the event that a sample of the neutralized formol is required for an external analysis, it will be taken from the cited sampling tap 16.

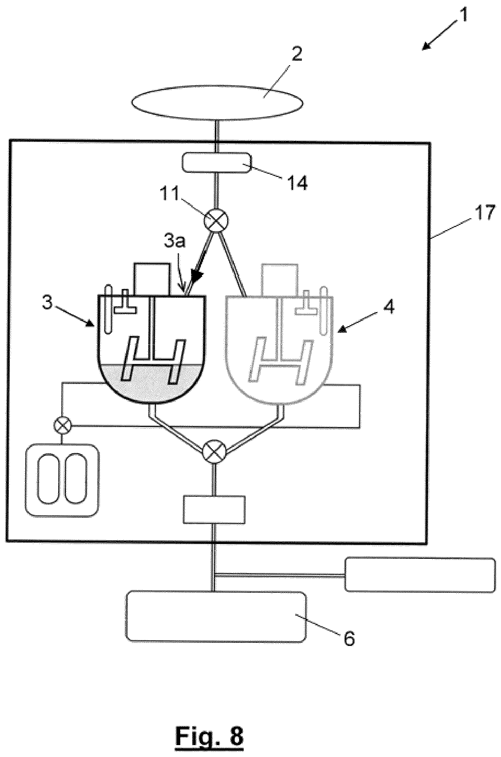

Once the drainage is carried out, as can be seen in Figure 8 (similar to Figure 2), the second reaction vessel 4 is empty in the inactive state, awaiting the first reaction vessel 3 to arrive at its maximum fill level.

The method is repeated successively such that the reaction vessels 3, 4 work alternately to thus guarantee the continuous operation of the system 1.

## Claims

1. A method for neutralizing formol, especially suitable for neutralizing formol used in centers for analyzing biological tissues, **characterized in that** it comprises the operations of:
a) supplying and storing quantities of formol to be neutralized associated with various uses or analyses in at least one reaction vessel (3, 4);
b) stopping the supply of formol to be neutralized when a maximum fill level of the formol content inside the reaction vessel (3, 4) has been reached in order to initiate the neutralization process;
c) supplying an ozone current inside the reaction vessel (3, 4) to produce a mixture with the formol content during a pre-established time to generate the neutralization process of the formol;
d) stopping the supply of the ozone current when the neutralization process of the formol has finished; and
e) draining the content of neutralized formol from the reaction vessel (3, 4) for its disposal.

2. The method for neutralizing formol according to claim 1, **characterized in that** it comprises the operation of stirring the formol content inside the reaction vessel (3, 4) while the ozone current is supplied to facilitate its mixing during the neutralization reaction.

3. The method for neutralizing formol according to claim 1 or 2, **characterized in that** it comprises the operation of measuring the pH of the formol and ozone mixture such that before carrying out the draining operation, it is verified whether the pH value of the neutralized formol is within standardized set values for decontamination of the formol.

4. The method for neutralizing formol according to any of the preceding claims, **characterized in that** the flow of formol to be neutralized is subjected to a solid filtering operation before being supplied to the reaction vessel (3, 4).

5. The method for neutralizing formol according to any one of the preceding claims, **characterized in that** the operation of supplying a flow of formol to be neutralized is carried out continuously by means of using two reaction vessels (3, 4) arranged in parallel which work in alternating cycles, such that while a first reaction vessel (3) is carrying out the neutralization reaction of the formol, the second reaction vessel (4) is storing formol to be neutralized and vice versa.

6. A system (1) for neutralizing formol, especially suitable for neutralizing formol used in centers for analyzing biological tissues, **characterized in that** it comprises:
- an intake (2) of the quantities of formol to be neutralized associated with various uses or analyses;
- at least one reaction vessel (3, 4) which comprises:
- an inlet (3a, 4a) of the flow of formol to be neutralized connected to said intake (2) of the system (1),
- a fill level detector (7) of the formol content to be neutralized inside the reaction vessel (3, 4), intended to send a signal to control means to initiate the neutralization process when a maximum fill level has been reached,
- a supply inlet (3b, 4b) of an ozone current, intended to produce a mixture with the formol content to generate the neutralization process of the formol,
- an outlet (3c, 4c) of the flow of neutralized formol;
- at least one ozone generator (5) equipped with an outlet (5a) for the injection of said ozone current towards the interior of the at least one reaction vessel (3, 4); and
- a drain (6) connected to the outlet (3c, 4c) of the at least one reaction vessel (3, 4) to remove the neutralized formol out of the system (1).

7. The system (1) for neutralizing formol according to claim 6, **characterized in that** it comprises two reaction vessels (3, 4) intended to work in alternating cycles, both vessels (3, 4) being connected in parallel to the intake (2), to the outlet (5a) of the ozone generator (5) and to the drain (6), by way of diverting conduits in the manner of bypasses, controlled by respective electrovalves (11, 12, 13).

8. The system (1) for neutralizing formol according to claim 6 or 7, **characterized in that** the reaction vessel (3), or when applicable each reaction vessel (3, 4), also comprises a motor (8) for actuating rotary blades (9) configured to stir the formol content inside the respective reaction vessel (3, 4) and facilitate its mixing with the ozone during the neutralization process.

9. The system (1) for neutralizing formol according to any one of claims 6 to 8, **characterized in that** the reaction vessel (3), or when applicable each reaction vessel (3, 4), also comprises a pH measurer intended to monitor the pH level of neutralized formol before proceeding to its drainage.

10. The system (1) for neutralizing formol according to any one of claims 6 to 9, **characterized in that** it comprises a filter (14) arranged before the reaction vessel (3), or when applicable the reaction vessels (3, 4), intended to prevent the accumulation of solid residues in its interior.

11. The system (1) for neutralizing formol according to any one of claims 6 to 10, **characterized in that** it comprises a pump (15) intended to force the outlet of the content of neutralized formol towards the drain (6).

12. The system (1) for neutralizing formol according to any one of claims 6 to 11, **characterized in that** it comprises a surrounding casing (17) configured to house in its interior all the components of the system (1) in a compact manner.

13. The system (1) for neutralizing formol according to any one of claims 6 to 12, **characterized in that** it comprises a sampling tap (16) arranged before the drain (6), intended to take samples of the neutralized formol for its analysis.

14. The system (1) for neutralizing formol according to any one of claims 6 to 13, **characterized in that** the drain (6) of the neutralized formol comprises means for its connection to the general sewerage network.

15. The system (1) for neutralizing formol according to any one of claims 6 to 14, **characterized in that** the intake (2) of the formol to be neutralized comprises means for its connection to an exterior conventional drain for collecting formol.
